(19) Europäisches Patentamt
European Patent Office
Office européen des brevets

(11) **EP 4 085 932 A1**

(12) **EUROPEAN PATENT APPLICATION**

(43) Date of publication:
**09.11.2022 Bulletin 2022/45**

(21) Application number: **21171830.9**

(22) Date of filing: **03.05.2021**

(51) International Patent Classification (IPC):
**A61K 48/00** (2006.01)   **C12N 15/11** (2006.01)
**C12N 15/85** (2006.01)

(52) Cooperative Patent Classification (CPC):
**C12N 15/67; A61K 31/7115; A61K 48/0066;
C12N 15/85**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **H.M.Z. Privatstiftung
8990 Bad Aussee (AT)**

(72) Inventors:
• **KORMANN, Michael
  71093 Weil im Schönbuch (DE)**

• **AKM Ashíqul, Haque
  72076 Tübingen (DE)**
• **WEIDENSEE, Brian
  72074 Tübingen (DE)**
• **SUMIT, Biswas
  72070 Tübingen (DE)**

(74) Representative: **Fuchs Patentanwälte
Partnerschaft mbB
Westhafenplatz 1
60327 Frankfurt am Main (DE)**

Remarks:
The complete document including Reference Table(s) and the Sequence Listing(s) can be downloaded from the EPO website

(54) **STABILIZED, MODIFIED RNA FOR USE IN THE TREATMENT OF A DISEASE ASSOCIATED WITH THE CYSTIC FIBROSIS TRANSMEMBRANE CONDUCTANCE REGULATOR (CFTR) GENE**

(57) The present invention relates to a stabilized chemically modified mRNA and to a method for the treatment of a disease associated with the *cystic fibrosis transmembrane conductance regulator* (*CFTR*) gene.

Figure 1

EP 4 085 932 A1

## Description

Field of the Invention

The present invention relates to a chemically modified mRNA and to a method for the treatment of a disease associated with the cystic fibrosis transmembrane conductance regulator (CFTR) gene.

Background of the Invention

A number of diseases is known in the art to be associated with the cystic fibrosis transmembrane conductance regulator (CFTR) gene. Such diseases include cystic fibrosis (CF), congenital absence of the vas deferens (CAVD) and chronic obstructive lung disease (COPD).

The *CFTR* gene codes for an ABC transporter-class ion channel protein that conducts chloride ions across epithelial cell membranes. Mutations of the *CFTR* gene affecting chloride ion channel function lead to dysregulation of epithelial fluid transport in the lung, pancreas and other organs, resulting in cystic fibrosis. Complications include thickened mucus in the lungs with frequent respiratory infections, and pancreatic insufficiency giving rise to malnutrition and diabetes. These conditions lead to chronic disability and reduced life expectancy. In male patients, the progressive obstruction and destruction of the developing *vas deferens* (spermatic cord) and epididymis appear to result from abnormal intraluminal secretions, causing congenital absence of the vas deferens and male infertility.

Cystic fibrosis (CF), the most common life-limiting genetic disease in Caucasian populations (1/2,500 newborns), affects more than 80,000 people world-wide. It is caused by the presence of mutations in both copies of the underlying disease conferring *CFTR* gene. Subjects with a single working copy are carriers, i.e. they can pass on the defective gene to the next generation, but are otherwise mostly normal. However, when both alleles of the *CFTR* gene are defective, the mutations result in an impaired anion secretion and hyper-absorption of sodium across epithelia. This again results an impairment of the production of sweat, digestive fluids, and mucus. In CF patients chronic lung disease and slow lung degradation is the major factor contributing to the mortality and morbidity in CF patients.

No cure for *cystic fibrosis* is known. Lung infections are treated with antibiotics which may be given intravenously, inhaled, or by mouth. Sometimes, the antibiotic azithromycin is used long term. Inhaled hypertonic saline and salbutamol may also be useful. Lung transplantation may be an option if lung function continues to worsen. With currently available therapies, which are very expensive, the mean survival is between 30-40 years. One of the most recent drugs is Ivacaftor, which is used to treat *cystic fibrosis* in people with certain mutations, primarily the G551D mutation, who account for 4-5% cases of cystic fibrosis. Ivacaftor is a substrate of *CYP3A4/5* and is one of the most expensive drugs, costing over US$ 300,000 per year.

The gene that encodes the human *CFTR* protein is found on chromosome 7, on the long arm at position q31.2. from base pair 116,907,253 to base pair 117,095,955. *CFTR* orthologs occur in the jawed vertebrates.

Since the *CFTR* gene was first cloned in 1989, several attempts have been made to correct the mutations at a cellular and genetic level. Gene therapy approaches aimed to deliver viral *CFTR* encoded vectors (such as adenoviruses (Ad) or adeno-associated viruses (AAV)) to CF patients. However, none of the clinical studies and current treatments seem to provide sufficient *hCFTR* expression to prevent the ultimately lethal CF symptoms in the airways of CF patients. Furthermore, repeated administration of viral vectors or DNA lead to the development of unwanted immune reactions, mainly due to viral capsids and vector-encoded proteins.

Congenital absence of the *vas deferens* (*CAVD*) is a condition in which the *vas deferens* reproductive organs, fail to form properly prior to birth. It may either be unilateral (*CUAVD*) or bilateral (*CBAVD*). The vas deferens connect the sperm-producing testicles to the penis. Therefore, those who are missing both *vas deferens* are typically able to create sperm, but are unable to transport them appropriately. Their semen does not contain sperm, a condition known as azoospermia. There are two main populations of *CAVD;* the larger group is associated with *cystic fibrosis* and occurs because of a mutation in the *CFTR* gene. Mutation of the *CFTR* gene is found to result in obstructive azoospermia in post-pubertal males with *cystic fibrosis.* Strikingly, *CAVD* is one of the most consistent features of cystic fibrosis as it affects up to 99% of individuals in this CF patient population. In contrast, acute or persistent respiratory symptoms present in only 51% of total CF patients.

Individuals with *CAVD* can reproduce with the assistance of modern technology with a combination of testicular sperm extraction and *intracytoplasmic sperm injection (ICSI)*. However, so far no causative therapy for *CAVD* is available. Also a surgical treatment is not possible.

*Chronic obstructive pulmonary disease* (*COPD*) is a type of obstructive lung disease characterized by long-term poor airflow. The main symptoms include shortness of breath and cough with sputum production. *COPD* is a progressive disease, meaning it typically worsens over time. Even though tobacco smoking is the most common cause of *COPD,* cases are known where mutations the *CFTR* gene were described as a disease-conferring cause.

Currently available *COPD* treatments include stopping smoking, vaccinations, respiratory rehabilitation, and often inhaled bronchodilators and steroids. However, therapeutic measures against forms of *COPD* resulting from a dysfunctional *CFTR* gene are so far not available.

Kormann et al. (2011), Expression of therapeutic proteins after delivery of chemically modified mRNA in mice, Letters to Nature Biotechnology, pages 1-6, describe a therapeutic approach for the treatment of SP-B deficiency where a functional nucleotide modified messenger RNA (mRNA) encoding SP-B is introduced into alveolar cells of the mouse.

Mahiny et al. (2015), In vivo genome editing using nuclease-encoding mRNA corrects SP-B deficiency, Nat. Biotechnology 33(6):584-586, and WO 2015/052133 disclose a nuclease-encoding nucleotide-modified messenger RNA (*nec-mRNA*) intended to correct a genetic alteration of the surfactant protein B (*SP-B*).

In WO 2018/202884 A1 (Kormann et al.) disclose a chemically modified mRNA and a method for the treatment of a disease associated with the *CFTR* gene. However, it could be shown that the stability of the chemically modified mRNAs disclosed therein is insufficient in order to be used as a therapeutic composition.

Against this background, the problem underlying the invention is to provide a new therapeutic approach and tool for the treatment of a disease associated with the *CFTR* gene which is highly effective, causative and implementable in the health system at reasonable costs. Further, with the new approach and tool the disadvantages of the prior art should be avoided or reduced, respectively.

The present invention satisfies these and other needs.

Detailed Description

In a first aspect the present invention pertains to a chemically modified cmRNA with an increased stability encoding a CF transmembrane conductance regulator or a derivative thereof (*CFTR cmRNA*), wherein said chemical modification is a replacement of non-modified nucleotides by pseudo-UTP (*ΨU*) and acetylated CTP (acC), and wherein at least 50% of the total number of UTP and CTP nucleotides is replaced by pseudo-UTP (*ΨU*) and acetylated CTP (acC).

It has been surprisingly found that the use of the combination of the two chemical modified nucleotides pseudo-UTP (*ΨU*) and acetylated CTP (acC) of at least 10% of the total nucleotides present in the mRNA, resulted in a synergistic effect of an increased stability and, as a result, in an improved expression of the reporter-gene, when the cmRNA is expressed transiently in a target cell.

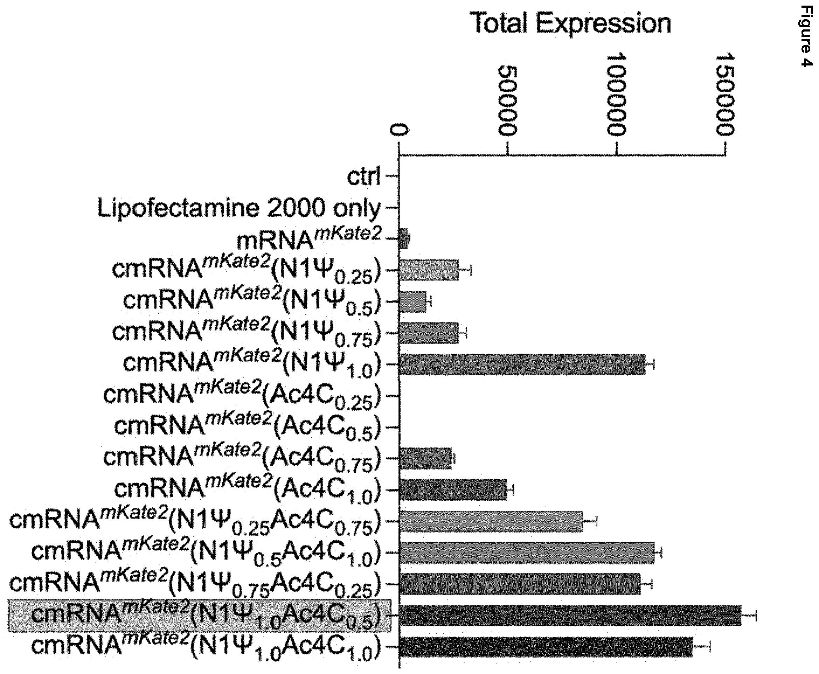

In some embodiments the highest stability is reached if at least approx. 50% and up to (including) approx. 100% of pseudo-UTP (*ΨU*) and at least approx. 50% up to (including) approx. 100% of acetylated CTP (acC) are combined; in one embodiment approx. 100% of pseudo-UTP (*ΨU*) and approx. 50% of acetylated CTP (acC) are combined to achieve best stability results (see figure 4).

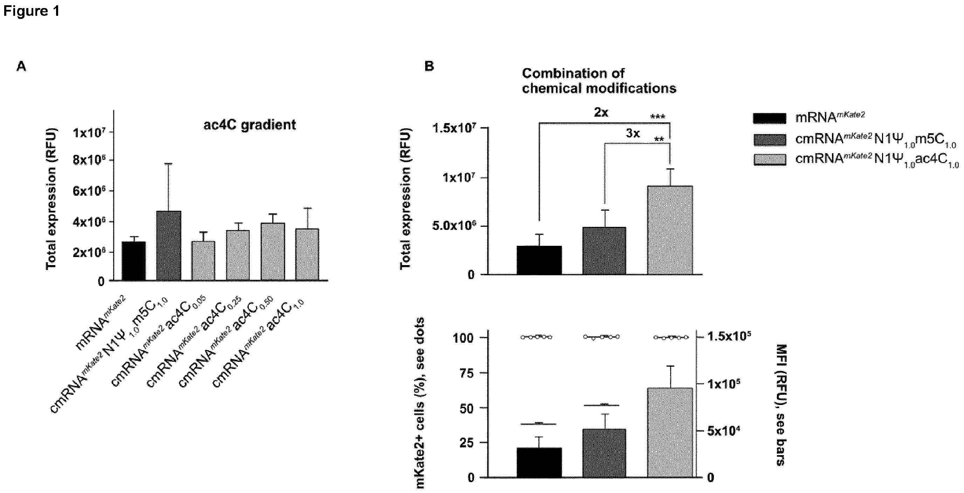

As such, the combination of the two chemical modified nucleotides pseudo-UTP (*ΨU*) and acetylated CTP (acC) is even superior in stability and expression profile as a combination of other modified nucleotides, for example the two chemical modified nucleotides pseudo-UTP (*ΨU*) and 5-methylated CTP (m5C) (see examples and figure 1b).

This is insofar surprising, since neither pseudo-UTP (*ΨU*) nor acetylated CTP (acC) alone do improve the stability and/or expression of a reporter gene significantly more as compared to other chemical modifications (such as for example 5-methylated CTP) (see examples and figures 1a and 4).

However, the combination of the two chemical modified nucleotides pseudo-UTP (*ΨU*) and acetylated CTP (acC) of at least 50% of the total nucleotides present in the mRNA resulted in at least 2 fold increase in expression-signal of the reporter gene (see examples and figure 1b).

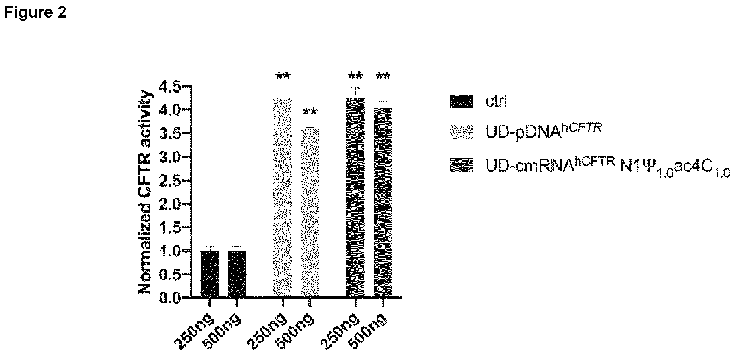

The stability of the cmRNA could be even further improved by Uridine-depletion (see examples and figure 2).

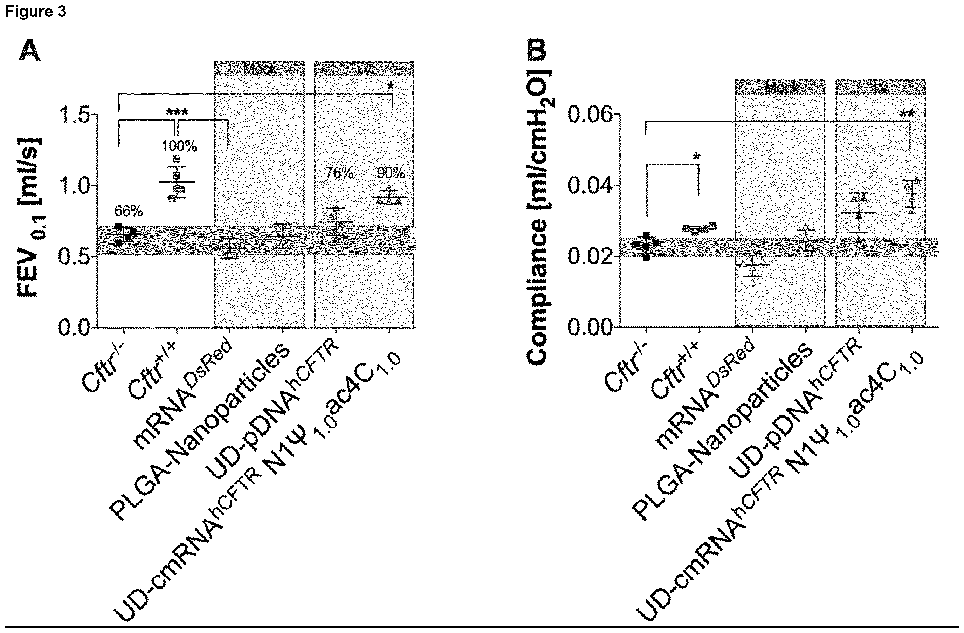

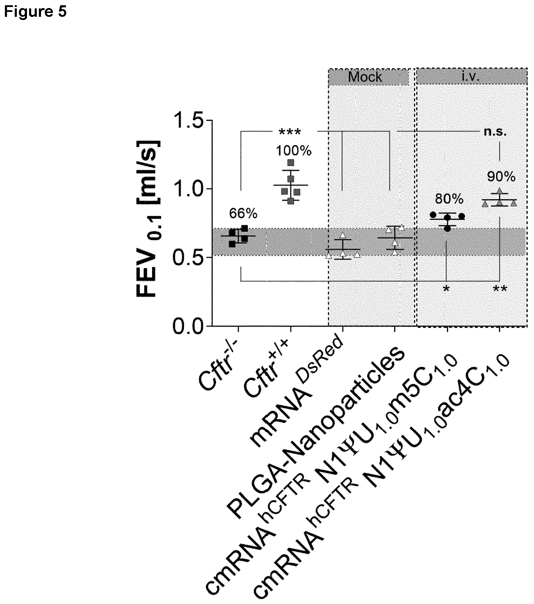

This surprising effect could also be shown for the expression of the CTFR-gene in vivo (mouse model), which resulted in a recovery of lung function of up to 90% as compared with wild-type mice (see examples and figures 3 and 5).

Thus, the present invention pertains to a new type of chemically modified cmRNA suitable for the treatment of defects in the CTFR-gene.

**Definitions**

According to the invention, *"chemically modified mRNA"* (*cmRNA*) refers to such an messenger ribonucleic acid, where at least a part of the nucleotides, or nucleosides or nucleobases is modified or changed with respect to their naturally occurring counterparts by the provision or omission of a chemical structure or entity. In this regard the terms *"nucleotides"* and *"nucleosides"* are used interchangeably. The chemical modification has the result that the cmRNA is more stable and has less immunogenicity. Nucleotide-modified messenger RNA is generally known in the prior art, cf. for example from WO 2011/012316. Examples for chemically-modified nucleotides or nucleosides are pseudouridine (*ΨU*), 5-methylcytidine (m5C), *N*6-methyladenosine (m6A), 5-methyluridine (m5U), 2-thiouridine (s2U) or acetylated CTP (acC).

In preferred embodiments the chemically-modified nucleotides or nucleosides which are used as part of the invention is a combination of a modified uridine which is pseudo-UTP (*ΨU*), preferably N1-methylpseudo-UTP (*meN1 ΨU*) and a modified cytidine which is acetylated CTP (acC), preferably N4-acetyl-CTP (ac4C).

According to the invention "CF *transmembrane conductance regulator"* (*CFTR*) refers to a functional membrane protein and chloride channel of vertebrates that is encoded by or derived from the CFTR gene of a vertebrate, e.g. a human (*hCFTR*) or animal being (*aCFTR*). The CFTR gene codes for an ABC transporter-class ion channel protein that conducts chloride and thiocyanate ions across epithelial cell membranes. Exemplary nucleotide and amino acid sequences (NCBI Reference Sequence; RefSeq) of CFTR are as follows: mRNA = NM_000492 (human) and NM_021050 (mouse); protein = NP_000483 (human) and NP_066388.1/NP_066388 (mouse). According to the invention the CFTR cmRNA may therefore comprise any of the before identified mRNA sequence or a nucleotide sequence encoding any of the before identified proteins.

According to an embodiment of the invention *"CFTR cmRNA"* includes not only a cmRNA encoding the entire CFTR gene product or comprising the full or identical coding sequence of the entire CFTR gene of a vertebrate, but also such a cmRNA encoding a derivative of the CFTR gene product. A *"derivative of CFTR"* according to the invention is a gene product, i.e. a protein or polypeptide which is still a physiologically and/or functionally active membrane protein and an ABC transporter-class ion channel that conducts chloride and thiocyanate ions across epithelial cell membranes.

A *"derivative of CFTR"* still has the biological activity of the wild type CFTR by at least 50%, further preferably by at least 60%, 70%, 80%, 90%, and highly preferably by 100%, as determined by a well-established functional test known to the skilled person.

A *"derivative of CFTR"* may comprise a section of the full length amino acid sequence of CFTR of a vertebrate, but also an amino acid sequence with a sequence homology to the full length amino acid sequence of CFTR of about at least approx. 90%, preferably of at least approx. 95%, 96%, 97%, 98%, 99% as determinable by BLAST; COBALT, or the like.

According to the invention the CFTR cmRNA includes both single- and double-stranded mRNA while, however, single-stranded mRNA is preferred.

According to the invention a "medicament" refers to a pharmaceutical composition wherein the CFTR cmRNA or the coding product is considered as the active agent. The CFTR cmRNA or the coding product may be the only active agent in the medicament. In an embodiment of the invention additional active agents may be provided. The medicament may include a pharmaceutically formulation comprising a carrier and, optionally, one or more accessory ingredients.

Depending on the way of administration the medicament may be present in a form appropriate for a delivery through the air ways of the patient, e.g. as an aerosol or a fine inhalable powder. Pharmaceutical formulations for oral administration can be formulated using pharmaceutically acceptable carriers well known in the art in appropriate and suitable dosages. Such carriers enable the pharmaceuticals to be formulated in unit dosage forms as tablets, pills, powder, dragees, capsules, liquids, lozenges, gels, syrups, slurries, suspensions, etc., suitable for ingestion by the patient.

In particular such capsules, tablet or pills are preferred which are acid resistant and allow the release of the CFTR cmRNA in the gastrointestinal tract. This may result in a restoration of the CFTR channels in the intestine and a release of the CFTR cmRNA into the blood stream.

In one embodiment an inhalable powder is produced by a nano spray dryer. The term "nano spray dryer" refers to devices enabling the spray drying of the formulation in order to create particles in the nanometer range. Spray drying is a gentle method for producing powders with a defined particle size out of solutions, dispersions, and emulsions which is widely used for pharmaceuticals, food, biotechnology, and other industrial materials synthesis.

In the past, the limitations of spray drying were the particle size (minimum 2 micrometers), the yield (maximum around 70%), and the sample volume (minimum 50 ml for devices in lab scale). Recently, minimum particle sizes have been reduced to 300 nm, yields up to 90% are possible, and the sample amount can be as small as 1 ml. These expanded limits are possible due to new technological developments to the spray head, the heating system, and the electrostatic particle collector. To emphasize the small particle sizes possible with this new technology, it has been described as "nano" spray drying. However, the smallest particles produced are in the sub-micrometer range common to fine particles rather than the nanometer scale of ultrafine particles.

In another embodiment of the invention the CFTR cmRNA is complexed or associated with a nanoparticle (NP), e.g. a PLGA-based NP, preferably at least partially chitosan-coated PLGA nanoparticles.

This formulation has the advantage that the absorption of the CFTR cmRNA into the cell or transfection efficiency, respectively, is significantly improved, in particular with respect to cells of the lung tissue, but also of other tissues.

In the sense of the invention *"nanoparticle"* refers to a particle between approx. 1 and approx. 300 nanometers in size (hydrodynamic diameter), preferably between approx. 50 nm and approx. 250 nm, further preferably between approx. 75 nm and approx. 200 nm, further preferably between approx. 100 nm and approx. 175 nm, and highly preferably between approx. 150 nm and approx. 160 nm.

The indicated sizes can be measured by methods generally known by the skilled person, e.g. after the nanoparticles are dispersed for 30 min in physiological saline or phosphate buffer, e.g. PBS. Well-established measurement techniques include transmission electron microscopy (*TEM*), scanning electron microscopy (*SEM*), atomic force microscopy (*AFM*), photon correlation spectroscopy (PCS), nanoparticle surface area monitor (*NSAM*), condensation particle counter (CPC), differential mobility analyzer, scanning mobility particle sizer (*SMPS*), nanoparticle tracking analysis (*NTA*), X-ray diffraction (*XRD*), aerosol time of flight mass spectroscopy, aerosol particle mass analyzer (*APM*). A preferred appropriate nanoparticle is based on polylactic co-glycolic acid (*PLGA*).

PLGA-based nanoparticles are e.g. described in Danhier et al. (2012), PLGA-based nanoparticles: An overview of biomedical applications, Journal of Controlled Release Vol. 161, Issue 2, p. 505-522.

Another example for an appropriate nanoparticle is lipid *GL67/DOPE.*

According to a preferred embodiment of the invention the nanoparticle is at least partially coated with chitosan.

This formulation has the advantage that the absorbability or respirability is further increased. In addition, chitosan has been proven as being particularly biocompatible resulting in an increase of the tolerance of the CFTR cmRNA by a living being.

As used herein, the term *"nucleic acid molecule"* refers to any nucleic acid containing molecule, including but not limited to DNA or RNA. Such a nucleic acid enables the targeted expression, replication and handling of CFTR or the CFTR mRNA.

The nucleic acid may comprise DNA, RNA, locked nucleic acid as well as PNA and it may be a hybrid thereof. The nucleic acid molecule of the present invention preferably is a recombinant nucleic acid molecule. It is evident to the person skilled in the art that regulatory sequences may be added to the nucleic acid molecule of the invention encoding the RNA molecule. For example, promoters, transcriptional enhancers, reporter genes, genes enabling the selection of a vector (i.e. antibiotic resistance) and/or sequences which allow for induced expression of the polynucleotide, i.e., the RNA molecule, of the invention may be employed.

Against this background another subject-matter of the present invention relates to a vector comprising said nucleic acid molecule, and a host cell comprising said vector.

The term *"vector"* as used herein refers to any nucleic acid molecule, preferably a DNA molecule, used as a vehicle to artificially carry said nucleic acid molecule encoding the CFTR cmRNA according to the invention into another cell, where it can be replicated and/or expressed. The vector of the present invention may be, e.g., a plasmid, cosmid, virus, bacteriophage or another vector used e.g. conventionally in genetic engineering, and may comprise further genes such as marker genes which allow for the selection of said vector in a suitable host cell and under suitable conditions.

Furthermore, the vector of the present invention may, in addition to the sequences of the nucleic acid molecule encoding the RNA molecule of the invention, comprise expression control elements, allowing proper expression of the coding regions in suitable hosts. Such control elements are known to the skilled person and may include a promoter, a splice cassette, translation start codon, translation and insertion site for introducing an insert into the vector. Preferably, the nucleic acid molecule of the invention is operatively linked to said expression control sequences allowing expression in eukaryotic or prokaryotic cells.

Accordingly, the present invention relates to a vector comprising the nucleic acid molecule of the present invention, wherein the nucleic acid molecule is operably linked to control sequences that are recognized by a host cell when the eukaryotic and/or prokaryotic (host) cell is transfected with the vector.

Furthermore, the vector of the present invention may also be an expression vector. The nucleic acid molecules and vectors of the invention may be designed for direct introduction or for introduction via liposomes, viral vectors (e.g. adenoviral, retroviral), electroporation, ballistic (e.g. gene gun) or other delivery systems into the cell. Additionally, a baculoviral system can be used as eukaryotic expression system for the nucleic acid molecules of the invention.

Thus, the present invention relates to a host transfected or transformed with the vector of the invention or a non-human host carrying the vector of the present invention, i.e. to a host cell or host which is genetically modified with a nucleic acid molecule according to the invention or with a vector comprising such a nucleic acid molecule. The transformation of the host cell with a vector according to the invention can be carried out by standard methods, as for instance described in Sambrook and Russell (2001), Molecular Cloning: A Laboratory Manual, CSH Press, Cold Spring Harbor, NY, USA; Methods in Yeast Genetics, A Laboratory Course Manual, Cold Spring Harbor Laboratory Press, 1990.

The host cell is cultured in nutrient media meeting the requirements of the particular host cell used, in particular in respect of the pH value, temperature, salt concentration, aeration, antibiotics, vitamins, trace elements etc.

As used herein *"host cell"* includes any biological cells of bacterial, fungal, animal, human or plant origin capable of receiving and, preferably, replicating and/or expressing the vector or CFTR cmRNA or CFTR, respectively. Examples for suitable fungal cells are yeast cells, preferably those of the genus Saccharomyces and most preferably those of the species Saccharomyces cerevisiae. Suitable animal cells are, for instance, insect cells, vertebrate cells, preferably mammalian cells, such as e.g. HEK293, NSO, CHO,COS-7, MDCK, U2-OSHela (HeLa), NIH3T3, MOLT-4, Jurkat, PC-12, PC-3, IMR, NT2N, Sk-n-sh, CaSki, C33A. Further suitable cell lines known in the art are obtainable from cell line depositories, like, e.g., the Deutsche Sammlung von Mikroorganismen und Zellkulturen GmbH (DSMZ) or the American Type Culture Collection (ATCC).

The features, characteristics and advantages of the CFTR cmRNA according to the invention apply likewise to the nucleic acid, the vector and the host cell according to the invention and vice versa.

The goal of the present invention is the transient expression of the CFTR-gene resulting in an intact CFTR-protein with correct functionality, thereby overcoming the defects of the CFTR-protein of the patient. Such a treatment most likely needs to be repeated in certain intervals, since the cmRNA is degraded after a while.

However, in other embodiments also an insertion of the cmRNA into the locus of the CFTR-target is encompassed. Such an insertion may most likely be facilitated outside the body, e.g. by extraction of stem cells and/or pluripotent cells and treatment of said cells with a composition comprising the cmRNA of the invention. Such gene-editing may be facilitated for example by reverse transkriptase enzymes and/or RT-viruses and/or by using the CRISPR/Cas9-mechanism. After such a successful gene-editing, the "corrected" cells may be transferred back into the patient and expand within the body.

According to a preferred embodiment of the invention the CFTR cmRNA is configured in a pharmaceutical composition for an inhalative (p.i.; *per inhalation*), i.e. an intratracheal (*i.t.*) and/or endobronchial (*e.b.*) and/or intrapulmonal (*i.pul.*) administration.

This formulation for such a method of administration has the advantage that the CFTR cmRNA is directly administered to the cells of interest in CF or COPD, i.e. the cells comprising the defective CFTR gene. The *i.t., e.b.* and/or *i.pul.* administration allows a contacting of the target cells directly in the respiratory tract where the CFTR dysfunction is causing damage. An appropriate configuration according to the invention includes an aerosol or an inhalable fine powder. The *i.t., e.b.* and/or *i.pul.* administration can e.g. be realized by means of a common microsprayer aerosolizer or a powder insufflator.

Thus, preferably, the CFTR cmRNA of the present invention is included into the pharmaceutical composition in an effective amount. The term *"effective amount"* refers to an amount sufficient to induce a detectable therapeutic response in the subject to which the pharmaceutical composition is to be administered. In accordance with the above, the content of the CFTR cmRNA of the present invention in the pharmaceutical composition is not limited as far as it is useful for treatment as described above, but preferably contains 0.01-10% by weight per total composition.

The features, characteristics and advantages of the *CFTR* cmRNA according to the invention apply likewise to the pharmaceutical composition according to the invention and vice versa.

The inventors have surprisingly recognized that chemically modified mRNA/polyribonucleotides with a specific ratio of uridine and cytidine modifications, especially with a combination of pseuodo-uridine and acetylcytidine, encoding the CF transmembrane conductance regulator (*CFTR cmRNA*) is a valuable tool, allowing the treatment of a disease or symptom associated with a defective or dysfunctional CFTR-gene or -protein, respectively.

In order to explain the cmRNA species, in certain embodiments the following nomenclature is used hereinunder:

$$\text{cmRNA}^{hCFTR}_{meN1\Psi \geq 0.95/ac_4C \geq 0.95}$$

This describes a chemically modified RNA (cmRNA) carrying the human CFTR-gene, wherein the modification is: 95% or more of the uridine nucleotides are N1-methylpseudo-uridine nucleotides and 95% or more of the cytidines are N4-acetyl-cytidine nucleotides.

In one embodiment 10% or more, more preferred 20% or more, even more preferred 30% or more, even more preferred 40% or more, even more preferred 50% or more, even more preferred 60% or more and up to 70% or less, more preferred up to 80% or less, even more preferred up to 90% or less, and even more preferred up to 100% or less of the uridines are modified uridines which is pseudo-UTP ( $\Psi U$ ), preferably N1-methylpseudo-UTP (*meN1 $\Psi U$*).

Thus, in some embodiments between 10% and 100%, in other embodiments between 25% and 100%, in yet other embodiments between 50% and 100%, in yet even other embodiments between 75% and 100%, in yet even other embodiments between 85% and 100%, in yet even other embodiments between 90% and 100%, in yet even other embodiments between 95% and 100% of the uridines are modified uridines which is pseudo-UTP ( $\Psi U$ ), preferably N1-methylpseudo-UTP (*meN1 $\Psi U$*).

The sequence of the cmRNA may be further optimized by *"uridine depletion"*. As it is well-known to the skilled person, three nucleotides (or a *"triplet"*) encode for one amino acid. In many cases not just one but several triplets encode the same amino acid, especially the third nucleotide of each triplet may be chosen between several nucleotides. Thus, in order to achieve a "uridine depletion" the wild-type triplets are analyzed *in silico* and are exchanged to ones which do not necessitate the use of uridine. Additionally in this process the abundance of certain tRNA within the target-cell is taken into account as well. Thereby reducing the number of uridine-nucleotides within the nucleic acid sequence and hence reducing the number of incorporated modified uridines within the cmRNA. A depletion of uridine (U) and the use of chemically-modified nucleotides may result in such a CFTR cmRNA which is not immunogenic without the need of HPLC purification. In a large scale waiving of HPLC purification significantly reduces the costs of the product according to the invention.

In one embodiment 10% or more, more preferred 20% or more, even more preferred 30% or more, even more preferred 40% or more, even more preferred 50% or more, even more preferred 60% or more, and up to 70% or less, more preferred up to 80% or less, even more preferred up to 90% or less, and even more preferred up to 100% or less of the cytidines are modified cytidines which is acetylated CTP (acC), preferably N4-acetyl-CTP (ac4C).

Thus, in some embodiments between 10% and 100%, in other embodiments between 25% and 100%, in yet other embodiments between 50% and 100%, in yet even other embodiments between 75% and 100%, in yet even other embodiments between 85% and 100%, in yet even other embodiments between 90% and 100%, in yet even other embodiments between 95% and 100% of the cytidines are modified cytidines which is acetylated CTP (acC), preferably N4-acetyl-CTP (ac4C).

In further embodiments the combination of the ratio between pseudo-uridine and acetylated cytidine is of importance. In one embodiment the pseudo-uridine is present within the cmRNA in at least 10% and up to 100% of all uridines within the sequence and the acetyl-cytidine is present within the cmRNA in at least 10% and up to 100% of all cytidines within the sequence; in further embodiments at least 25% and up to 100% pseudo-uridine of all uridines and at least 25% and up to 100% acetyl-cytidine of all cytidines are present within the sequence; in further embodiments at least 50% and up to 100% pseudo-uridine of all uridines and at least 50% and up to 100% acetyl-cytidine of all cytidines are present within the sequence; in further embodiments at least 75% and up to 100% pseudo-uridine of all uridines and at least 75% and up to 100% acetyl-cytidine of all cytidines are present within the sequence; in further embodiments at least 95% and up to 100% pseudo-uridine of all uridines and at least 95% and up to 100% acetyl-cytidine of all cytidines are present within the sequence; wherein in all before mentioned embodiments as pseudo-uridine N1-methylpseudo-UTP (*meN1ΨU*) is preferred and as acetyl-cytidine N4-acetyl-CTP (ac4C) is preferred.

The ratio between pseudo-uridine, preferably N1-methylpseudo-UTP (*meN1ΨU*), and acetyl-cytidine, preferably acetyl-cytidine N4-acetyl-CTP (ac4C), may also be taken into account. In preferred embodiments the ratio is between 1:10 and 10:1. That means, if for example the ratio is 1:10, for each pseudo-uridine within the sequence, the sequence comprises at least ten acetyl-cytidines. Further preferred ratios are between 1:5 and 5:1, preferably between 1:3 and 3:1, and even more preferred between 1:2 and 2:1, in some embodiments the ratio may be 1:1.

Surprisingly, the invention can be successfully used independent of the underlying *CFTR* dysfunction or mutation, respectively.

The inventors were able to demonstrate not only *in vitro,* but also *in vivo* in a well-established mouse model of CFTR-associated diseases that the administration of the *CFTR* cmRNA according to the invention, either intratracheally (i.t.) but also intravenously (i.v.), results in a drastically increase of the most important cellular parameters in comparison with untreated mice.

Thus, in one embodiment the *in-vivo* treatment a CFTR$^{-/-}$-mouse with cmRNA$^{hCFTR}_{meN1Ψ1.0/ac_4C1.0}$ resulted in 90% of the forced expiratory pressure in 0.1 second (FEV$_{0.1}$) of a wild-type (i.e. CFTR$^{+/+}$) mouse (cf. examples and figure 3 A).

Furthermore, also the compliance of the lungs and the thorax is significantly improved, reaching nearly normal levels (cf. examples and figure 3 B). The "compliance of the lungs and the thorax" is a measure of the flexibility of the respiratory system or its components and is defined as the ratio of volume change to the associated pressure change. Lung extensibility is an important means of assessing the integrity of the lung tissue and the compliance of the entire lung-thoracic system to control ventilation therapy.

In fact, as exemplified by lung parameters, the parameters are normalized by the treatment with the *CFTR* cmRNA according to the invention.

Thus, in one embodiment the invention pertains to aspects where about 24h after treatment with the inventive cmRNA the FEV$_{1.0}$ (in humans) is at least 80%, preferably at least 85%, even more preferably 90% or more of the mean FEV$_{1.0}$ of subjects of the same age, gender and overall constitution without a defect in the CFTR-gene.

Thus, in yet another embodiment the invention pertains to aspects where about 24h after treatment with the inventive cmRNA the compliance is significant increased and comparable to subjects of the same age, gender and overall constitution without a defect in the CFTR-gene.

The inventors were also able to demonstrate the functioning of the *CFTR* protein encoded by the *CFTR* cmRNA in the transfected cells, both *in vitro* and *in vivo.* Importantly, even a repeated administration of the *CFTR* cmRNA according to the invention does not result in an immune response as demonstrated by the inventors in different scientifically well accepted immunoassays.

Another subject-matter of the invention relates to a method for the treatment of a disease associated with the CFTR gene, comprising the following steps:

1. (optional) screening a living being (preferably a human) in order to identify the defect in the CFTR-gene;

2. providing the pharmaceutical composition according to the invention, which is (optional) adapted to the specific needs of the living being (preferably a human) with said specific CFTR-defect; and

3. administering said pharmaceutical composition into a living being, wherein an inhalative treatment is preferred;

4. (optional) repeating step 3 in suitable intervals so that a steady-state expression of the non-defective CFTR-gene product is reached within the living being (preferably a human).

In the present invention, the living being is, in a preferred embodiment, a mammal such as a dog, cat, pig, cow, sheep, horse, rodent, e.g., rat, mouse, and guinea pig, or a primate, e.g., gorilla, chimpanzee, and human. In a most preferable embodiment, the subject is a human.

Above mentioned screening may be done by biomarker-screening, PCR-analysis of the CFTR-gene and other methods known to the skilled person.

The above mentioned intervals may be chosen first to be about between 24 hours and about 72 hours between each treatment and may be adjusted later depending on the improvement of symptoms; i.e. if the effect of improvement and/or the levels of functional CFTR-gene product is too low, the interval may be decreased down to about 18 hours, down to about 12 hours, down to about 6 hours; whereas if the effect of improvement and/or the levels of functional CFTR-gene product is good or even high, than the interval may be increased up to about 48 hours, up to about 72 hours, up to about 84 hours and even up to about 96 hours.

The features, characteristics and advantages of the CFTR cmRNA according to the invention apply likewise to the method according to the invention and vice versa.

The present invention also relates to a kit comprising the CFTR cmRNA molecule of the present invention, the nucleic acid molecule of the present invention, the vector of the present invention or the host cell of the present invention. As regards the preferred embodiments, the same applies, mutatis mutandis, as has been set forth above in the context of the CFTR cmRNA molecule, nucleic acid molecule, vector or the host cell according to the present invention. Advantageously, the kit of the present invention further comprises, optionally (a) buffer(s), storage solutions and/or remaining reagents or materials required for the conduct of the above and below uses and methods.

Furthermore, parts of the kit of the invention can be packaged individually in vials or bottles or in combination in containers or multicontainer units. The kit of the present invention may be advantageously used, inter alia, for carrying out the method of the invention, the preparation of the CFTR cmRNA molecule of the invention and could be employed in a variety of applications referred herein, e.g., in the uses as outlined above and below. Another component that can be included in the kit is instructions to a person using a kit for its use.

Furthermore, devices for the application of the pharmacological composition of the invention are encompassed, such as inhalative devices, such as nebulizers, pressurized metered-dose inhaler (pMDI), and dry powder inhalers (DPIs). In case of i.v.-applications devices such as auto-injectors, pre-filled syringes, pump-devices and/or catheters or implantable ports may be used. Since a repeated treatment with the inventive pharmaceutical compositions is envisaged, an automated pump device which can deploy several dosages over a given time-period is preferred.

In one aspect the present invention pertains to chemically modified mRNA *(cmRNA)* with an increased stability encoding a *cystic fibrosis transmembrane conductance regulator* (*CFTR*), or a derivative thereof; wherein said chemical modification is a replacement of at least approx. 10%; preferably of at least approx. 15%; more preferably of at least approx. 25%; even more preferably of at least approx. 50% and up to approx. 75%, preferably up to approx. 90%, more preferably up to approx. 95%, even more preferably up to approx. 100% of the total number of UTP and CTP nucleotides with pseudo-UTP ($\Psi U$) and acetylated CTP (acC).

In another aspect the present invention pertains to chemically modified mRNA, wherein the increased stability results in an increased expression of the CFTR gene-product in a cell.

In one embodiment the expression is increased by a factor of 1.5 or more, preferably by a factor of 2 or more, even more preferably by a factor of 2.5 or more, even more preferably by a factor of 3 or more as compared to the expression of a unmodified mRNA with the same CFTR gene-product in a cell.

In yet another aspect the present invention pertains to chemically modified mRNA, wherein the CFTR cmRNA is complexed with a nanoparticle, preferably a polylactic-coglycolic acid (*PLGA*) based nanoparticle, preferably at least partially coated with chitosan.

In yet another aspect the present invention pertains to chemically modified mRNA configured for inhalative (*p.i.*) administration or an intravenous (*i.v.*) administration.

In yet another aspect the present invention pertains to chemically modified mRNA, wherein said CF trans-membrane conductance regulator is of human origin (hCFTR).

In yet another aspect the present invention pertains to chemically modified mRNA, wherein at least approx. 25 %, preferably at least approx.50% and preferably up to including approx. 75 %, further preferably up to including approx. 95 %, further preferably up to including approx. 100 % of the uridine nucleotides are replaced, wherein the uridine is replaced with pseudo-UTP ($\Psi U$), most preferably with N1-methylpseudo-UTP (meN1$\Psi U$).

In yet another aspect the present invention pertains to chemically modified mRNA, wherein at least approx. 25 %, preferably at least approx.50% and preferably up to including approx. 75 %, further preferably up to including approx. 95 %, further preferably up to including approx. 100 % of the cytidine nucleotides are replaced, wherein the cytidine is replaced with acetylated CTP (acC), most preferably with N4-acetyl-CTP (ac4C).

In yet another aspect the present invention pertains to chemically modified mRNA, wherein at least approx. 25 % of uridine and at least approx. 25 % of cytidine; more preferably at least approx. 50 % of uridine and at least approx. 50 % of cytidine; more preferably at least approx. 75 % of uridine and at least approx. 75 % of cytidine; more preferably at least approx. 95 % of uridine and at least approx. 95 % of cytidine; most preferably of up to approx. 100 % of uridine and up to approx. 100 % of cytidine are replaced, wherein the uridine is replaced with pseudo-UTP (ΨU), most preferably with N1-methylpseudo-UTP (meN14ΨU) and the cytidine is replaced with acetylated CTP (acC), most preferably with N4-acetyl-CTP (ac4C).

In yet another aspect the present invention pertains to chemically modified mRNA, wherein at least approx. 50 % of uridine and at least approx. 25 % of cytidine; more preferably at least approx. 75 % of uridine and at least approx. 25 % of cytidine; more preferably at least approx. 95 % of uridine and at least approx. 25 % of cytidine; more preferably at least approx. 95 % of uridine and at least approx. 25 % of cytidine; most preferably of up to approx. 100 % of uridine and up to approx. 25 % of cytidine are replaced, wherein the uridine is replaced with pseudo-UTP (ΨU), most preferably with N1-methylpseudo-UTP (meN1ΨU) and the cytidine is replaced with acetylated CTP (acC), most preferably with N4-acetyl-CTP (ac4C).

In yet another aspect the present invention pertains to chemically modified mRNA, wherein at least approx. 50 % of uridine and at least approx. 50% of cytidine; more preferably at least approx. 75 % of uridine and at least approx. 50 % of cytidine; more preferably at least approx. 95 % of uridine and at least approx. 50 % of cytidine; most preferably of up to approx. 100 % of uridine and up to approx. 50 % of cytidine are replaced, wherein the uridine is replaced with pseudo-UTP (ΨU), most preferably with N1-methylpseudo-UTP (meN1ΨU) and the cytidine is replaced with acetylated CTP (acC), most preferably with N4-acetyl-CTP (ac4C).

In yet another aspect the present invention pertains to chemically modified mRNA, wherein approx. 25 % to approx. 100% of uridine and at least approx. 25% to approx. 100% of cytidine; more preferably at least approx. 50 % to approx. 100% of uridine and at least approx. 50 % to approx. 100% of cytidine; more preferably at least approx. 75 % to approx. 100% of uridine and at least approx. 75 % to approx. 100% of cytidine; most preferably of up to approx. 95 % to approx. 100% of uridine and up to approx. 95 % to approx. 100% of cytidine are replaced, wherein the uridine is replaced with pseudo-UTP (ΨU), most preferably with N1-methylpseudo-UTP (meN1ΨU) and the cytidine is replaced with acetylated CTP (acC), most preferably with N4-acetyl-CTP (ac4C).

In yet another aspect the present invention pertains to chemically modified mRNA, wherein approx. 25 % to approx. 95% of uridine and at least approx. 25% to approx. 95% of cytidine; more preferably at least approx. 50 % to approx. 90% of uridine and at least approx. 50 % to approx. 90% of cytidine; more preferably at least approx. 75 % to approx. 85% of uridine and at least approx. 75 % to approx. 85% of cytidine are replaced, wherein the uridine is replaced with pseudo-UTP (ΨU), most preferably with N1-methylpseudo-UTP (meN1ΨU) and the cytidine is replaced with acetylated CTP (acC), most preferably with N4-acetyl-CTP (ac4C).

In yet another aspect the present invention pertains to chemically modified mRNA, wherein approx. 50 % to approx. 95% of uridine and at least approx. 50% to approx. 95% of cytidine; more preferably at least approx. 50 % to approx. 90% of uridine and at least approx. 50 % to approx. 90% of cytidine; more preferably at least approx. 50 % to approx. 85% of uridine and at least approx. 50 % to approx. 85% of cytidine are replaced, wherein the uridine is replaced with pseudo-UTP (ΨU), most preferably with N1-methylpseudo-UTP (meN1ΨU) and the cytidine is replaced with acetylated CTP (acC), most preferably with N4-acetyl-CTP (ac4C).

In yet another aspect the present invention pertains to chemically modified mRNA, wherein at least approx. 50% of UTP was replaced with N1-methylpseudo-UTP and at least approx. 50% of CTP was replaced N4-acetyl-CTP resulting in

$$cmRNA^{hCFTR}_{meN1\Psi\geq0.5/ac4C\geq0.5}.$$

In yet another aspect the present invention pertains to chemically modified mRNA, wherein at least approx. 95% of UTP was replaced with N1-methylpseudo-UTP and at least approx. 95% of CTP was replaced N4-acetyl-CTP resulting in

$$cmRNA^{hCFTR}_{meN1\Psi\geq0.95/ac4C\geq0.95}.$$

In yet another aspect the present invention pertains to chemically modified mRNA, wherein between 20% and 30%; preferably at least approx. 25% of UTP was replaced with N1-methylpseudo-UTP and wherein between 70% and 80%; preferably at least approx. 75% of CTP was replaced N4-acetyl-CTP resulting in

$$cmRNA^{hCFTR}_{meN1\Psi\geq0.25/ac4C\geq0.75}.$$ Said combination resulted in an improved expression profile in cell-culture as compared to other cmRNAs (see figure 4).

In yet another aspect the present invention pertains to chemically modified mRNA, wherein between 70% and 80%; preferably at least approx. 75% of UTP was replaced with N1-methylpseudo-UTP and between 20% and 30%; preferably at least approx. 25% of CTP was replaced N4-acetyl-CTP resulting in

$$cmRNA^{hCFTR}_{meN1\Psi\geq0.75/ac4C\geq0.25}.$$ Said combination resulted in an improved expression profile in cell-culture as compared to other cmRNAs (see figure 4).

In yet another aspect the present invention pertains to chemically modified mRNA, wherein between 45% and 55%; preferably at least approx. 50% of UTP was replaced with N1-methylpseudo-UTP and wherein approx. 100% of CTP was replaced N4-acetyl-CTP resulting in $cmRNA^{hCFTR}_{meN1\Psi\geq0.5/ac4C1.0}$. Said combination resulted in an improved expression profile in cell-culture as compared to other cmRNAs (see figure 4).

In yet another aspect the present invention pertains to chemically modified mRNA, wherein approx.100% of UTP was replaced with N1-methylpseudo-UTP and approx. 100% of CTP was replaced N4-acetyl-CTP resulting in $cmRNA^{hCFTR}_{meN1\Psi1.0/ac4C1.0}$. Said combination resulted in the second highest expression profile in cell-culture (see figure 4).

In yet another aspect the present invention pertains to chemically modified mRNA, wherein approx. 100% of UTP was replaced with N1-methylpseudo-UTP and wherein between 45% and 55%; preferably at least approx. 50% of CTP was replaced N4-acetyl-CTP resulting in $cmRNA^{hCFTR}_{meN1\Psi\geq1.0/ac4C1.0}$. Surprisingly said combination resulted in the highest expression profile in cell-culture (see figure 4).

In yet another aspect the present invention pertains to chemically modified mRNA of any of the preceding claims, wherein the expression profile is increased *in vitro* by at least a factor of 1.0, preferably by at least a factor of 1.50, preferably by at least a factor of 2.00, preferably by at least a factor of 2.50, preferably by at least a factor of 3.00, preferably by at least a factor of 3.50 as compared to the expression of an unmodified CFTR mRNA as control; and/or wherein the expression profile is increased *in vitro* by at least a factor of 1.05, preferably by at least a factor of 1.15, preferably by at least a factor of 1.25, preferably by at least a factor of 1.5, preferably by at least a factor of 2.0 as compared to the expression of a modified CFTR cmRNA as control, with the same amount of modified UTPs and CTPs, wherein the control CFTR cmRNA comprises a methyl-CTP, such as 5-methyl-CTP.

In yet another aspect the present invention pertains to chemically modified mRNA, wherein the expression profile is increased *in vitro* by at least a factor of 1.5, preferably by at least a factor of 2, preferably by at least a factor of 3, preferably by at least a factor of 4, preferably by at least a factor of 5 as compared to the expression of an unmodified CFTR mRNA as control; and/or wherein the expression is increased in vitro by at least a factor of 1.25, preferably by at least a factor of 1.5, preferably by at least a factor of 2.0, preferably by at least a factor of 2.5 as compared to the expression of cmRNA with the same amount of modified UTPs and CTPs, wherein the control CFTR cmRNA comprises a methyl-CTP, such as 5-methyl-CTP ; and/or wherein the expression of CFTR $cmRNA^{hCFTR}_{meN1\Psi1.0/ac4C1.0}$ is increased *in vitro* by at least a factor of 1.5, preferably by at least a factor of 2, preferably by at least a factor of 3, preferably by at least a factor of 4 as compared to the expression of $cmRNA^{hCFTR}_{meN1\Psi1.0/5mC1.0}$.

In yet another aspect the present invention pertains to chemically modified mRNA, wherein the CFTR cmRNA is further optimized by uridine-depletion, resulting in the increase of the expression profile in vitro by at least a factor of 2, preferably by at least a factor of 3, most preferably by a factor of 4 as compared to the expression of an unmodified CFTR mRNA as control.

In yet another aspect the present invention pertains to chemically modified mRNA of any of the preceding claims, wherein 24 h after treatment of the CTFR-deficient mice with said chemically modified mRNA the Forced Expiratory Volume in 0.1 second ($FEV_{0.1}$) in CTFR-deficient mice is at least 75%, preferably is at least 80%, even more preferably is at least 85%, most preferably is at least 90% or more as compared with the $FEV_{0.1}$ of control group consisting of wild-type mice treated with placebo.

In one embodiment 24 h after treatment of the CTFR-deficient mice with said chemically modified mRNA the Forced Expiratory Volume in 0.1 second ($FEV_{0.1}$) in CTFR-deficient mice is 100% as compared with the $FEV_{0.1}$ of control group consisting of wild-type mice treated with placebo. In one embodiment the Forced Expiratory Volume in CTFR-deficient mice treated with the cmRNAs of the present invention is even better than the control group consisting of wild-type mice treated with placebo.

In one embodiment 24 h after treatment of a CF-patient with said chemically modified mRNA the Forced Expiratory Volume in 1 second ($FEV_{1.0}$) in the CF-patient is at least 75%, preferably is at least 80%, even more preferably is at least 85%, more preferably is at least 90% or most preferably is 100% as compared with the $FEV_{1.0}$ of a healthy control group without defect in the CFTR-gene.

In yet another aspect the present invention pertains to chemically modified mRNA of the present invention for use as a medicament in humans and animals.

In yet another aspect the present invention pertains to chemically modified mRNA of the present invention for use in the treatment of a human subject or animal having or suspected of having a disease associated with the CFTR gene, preferably selected from the group consisting of: cystic fibrosis (CF), congenital absence of the vas deferens (CAVD) and/or chronic obstructive lung disease (COPD).

In yet another aspect the present invention pertains to a pharmaceutical composition comprising the chemically modified mRNA *(cmRNA)* according to the present invention and further comprising a pharmaceutically acceptable excipient and/or carrier and/or diluents.

In yet another aspect the present invention pertains to the use of a pharmaceutical composition comprising the chemically modified mRNA *(cmRNA)* according to the present invention for the treatment of a disease associated with the CFTR gene; optionally further comprising a pharmaceutically acceptable excipient and/or carrier and/or diluents.

In yet another aspect the present invention pertains to a host cell comprising the chemically modified mRNA of the present invention.

In yet another aspect the present invention pertains to the use of a pharmaceutical composition comprising the host cell comprising the chemically modified mRNA of the present invention for the treatment of a disease associated with the CFTR gene; optionally further comprising a pharmaceutically acceptable excipient and/or carrier and/or diluents.

The term "pharmaceutically acceptable excipient" encompasses any substance which has no (or only little) medicinal properties on its own, but its purpose is to streamline the manufacture of the drug product and ultimately facilitate physiological absorption of the drug. Such "pharmaceutically acceptable excipients" might aid in lubricity, flowability, disintegration, taste and may confer some form of antimicrobial function. An excipient must best suit the intended dosage form of the drug, demonstrate great organoleptic properties, conform to pharmacopeial regulations, be easy to source, and work effectively. The right excipient will have the ideal pharmacokinetic properties for the intended pharmaceutical application. Other considerations include potential toxicity, the origin of the chemicals, and other special factors. For example, a plant-based excipient might be chosen over one of animal origin (gelatine).

For example such "excipients" may be binder excipients, carrier excipients, co-processed excipients, coating systems excipients, controlled release excipients, diluent excipients, disintegrant excipients, dry powder inhalation excipients, effervescent system excipients, emulsifier excipients, film former excipients, lipid excipients, lubricant excipients, modified release excipients, penetration enhancer excipients, permeation enhancer excipients, pH modifier excipients, plasticizer excipients, preservative excipients, solubilizer excipients, solvent excipients, surfactant excipients, sustained release excipients, sweetener excipients, taste masking excipients, thickener excipients, viscosity modifier excipients, filler excipients, natural origin excipients, compaction excipients, direct compression excipients, dry granulation excipients, hot melt extrusion excipients, wet granulation excipients, rapid release agent excipients, increased bioavailability excipients, dispersion excipients, solubility enhancement excipients, stabilizer excipients, multifunctional excipients, granulation excipients, capsule filling excipients, powder blends excipients and/or tablet compressibility excipients.

In yet another aspect the present invention pertains to a method wherein CFTR-defective cells (i.e. for example lung cells, organ cells and/or pluripotent or omnipotent stem cells) are isolated from a patient, treated extra-corporal with the chemically modified mRNA of the present invention and transferred back to the patient in order to treat a disease associated with a defect in the CFTR-gene.

Short description of the figures

The invention is also described and explained in further detail by referring to the following drawings:

Figure 1 Total expression of 500ng *mKate2* mRNA and chemically modified derivatives (calculated by multiplying positive cells with their median fluorescence intensity) in A549 cells.

Figure 2 Quenching efficacy of 250ng or 500ng UD-pDNA$^{hCFTR}$ or UD-cmRNA$^{hCFTR}_{meN1\Psi1.0/ac4C1.0}$ transfected CFBE41o- cells relative to non-transfected controls was measured at 24 h post-transfection. $**P \leq 0.01$ versus non-transfected controls

Figure 3 *In vivo* lung function measurements in mRNA$^{mKate2}$, PLGA-nanoparticles, UD-pDNA$^{hCFTR}$ and UD-cmRNA$^{hCFTR}_{meN1\Psi1.0/ac4C1.0}$ treated *Cftr$^{-/-}$* mice by i.v. route. Precision *in vivo* lung function measurements covering all relevant outcome parameters. Data represents the means +/- SD on Forced Expiratory Volume in 0.1 seconds (FEV$_{0.1}$) and compliance. $*P \leq 0.05$; $**P < 0.01$ and $***P < 0.001$ versus untreated *Cftr$^{-/-}$* mice.

Figure 4 Total expression of 500ng *mKate2* mRNA and chemically modified derivatives (calculated by multiplying positive cells with their median fluorescence intensity) in A549 cells. The combination of N1-methylpseudo-UTP (N1ΨU) and N4-acetyl-CTP (ac4C) showed the best expression results. Interestingly the combination of 100% N1ΨU with 50% ac4C showed slightly better results than both modifications at 100%.

Figure 5 Another *in vivo* lung function measurement in mRNA$^{mKate2}$, PLGA-Nanoparticles, UD-pDNA$^{hCFTR}$ and

UD-cmRNA$^{hCFTR}_{meN1\Psi1.0/ac4C1.0}$ treated

*Cftr$^{-/-}$* mice by i.v. route. Precision *in vivo* lung function measurements covering all relevant outcome parameters. Data represents the means +/- SD on Forced Expiratory Volume in 0.1 seconds (FEV$_{0.1}$) and compliance. *$P \leq 0.05$; **$P < 0.01$ and ***$P < 0.001$ versus untreated *Cftr$^{-/-}$* mice.

## Examples

### Example 1: mRNA production

hCFTR was PCR amplified from pcDNA3.hCFTR with primers adding *Nhe*I (Fwd: 5'-TTAGCTA-GATGCAGAGGTCGCCTC-3') and *Kpn*I (Rev: 5'- GCGGGTACCTATCTTGCATCTCTTCT -3') restriction sites to each end. The PCR product was cloned into a poly(A)-120 containing pVAX (pVAX.A120, www.lifetechnologies.com) by sticky-end ligation using the mentioned restrictionsites. pVAX.A120 containing hCFTR is referred as pDNA hCFTR throughout this study. For control experiments, mKate2 reporter protein was sub-cloned into pVAX.A120 vector from its original vector pmKate2 (www.clontech.com). For *in vitro* transcription (IVT), the plasmids were linearized downstream of the poly(A) tail with *Xho*I (www.neb.com). IVT reaction was carried out using MEGAscript T7 Transcription kit (www.ambion.com) with an anti-reverse CAP analog (ARCA) at the 5' end (www.trilink.com). To produce modified mRNAs, the following chemically modified nucleosides were added to the IVT reaction in the indicated ratios: 25%/100% N1-methylpseudo-UTP, and 25%/100% N4-acetyl-CTP (www.trilink.com). The hCFTR and DsRed mRNA were purified using the MEGAclear kit (www.ambion.com) and analyzed for size and concentration using an Agilent 2100 Bioanalyzer (www.agilent.com).

### Example 2: Mammalian cell culture and transfection

A549 cells, human bronchial epithelial (HBE) and cystic fibrosis epithelial (CFBE) cell lines were maintained in Minimum Essential Medium (MEM, www.biochrom.com) supplemented with 10% (v/v) heat-inactivated Foetal Calf Serum (FCS), L-Glutamine (2mM) and Penicillin -Streptomycin (50U/ml). Cells were incubated at 37°C in a humidified atmosphere containing 5% CO$_2$ until they reached 80-90% confluency. Cell lines were washed with cold sterile PBS and detached by trypsin-EDTA. Trypsination was stopped by adding MEM medium with serum. Cells were collected and spun down at 500 x g for 5 minutes before resuspension in fresh MEM.

One day before transfection 250,000 cells/well/1ml were plated in 12 well plates and grown overnight in MEM without antibiotics. At a confluency of 70-90%, cells were then transfected with 1000 ng mRNA encoding hCFTR using lipofectamine 2000 (www.invitrogen.com) following the manufacturer's instructions and after changing the media to the reduced serum media, Opti-MEM (www.thermofisher.com). After 5 h, cells were washed with PBS and serum-containing MEM was added. Cells were kept for 24 h and 72 h before further analyses.

### Example 3: Flow cytometry analyses

All flow cytometry analyses were performed using a BD Fortessa X-20 SORP (www.bdbioscience.com). For detection of hCFTR protein in HBE and CFBE cell lines, cells were transfected as described above and subsequently prepared for intracellular staining using a Fixation/Permeabilization Solution Kit as directed in the manufacturer's instruction (www.bdbioscience.com). As primary antibody mouse anti-human hCFTR clone 596 (1:500, kindly provided by the cystic fibrosis foundation therapeutics Inc.) has been used. As secondary antibody served Alexa Fluor 488 goat anti-mouse IgG (1:1,000, www.lifetechnologies.com). At least 20,000 gated cells per tube were counted. Data were analyzed with the FlowJo software, version 10.

### Example 4: YFP-based functional assay

CFTR activity following transient transfection of CFTR mRNAs in A549 cells was determined using the halide-sensitive yellow fluorescent protein YFPH148Q/I152L (Galietta et al., 2001) as also described previously (Caputo et al., 2009).

A549 cells stably expressing the YFP were plated in 96-well microplates (50,000 cells/well) in 100 μl of antibiotic-free culture medium and, after 6 h, transfected with either plasmids carrying the coding sequence for CFTR or different CFTR mRNAs.

For each well, 0.25 or 0.5 μg of mRNA or plasmid DNA and 0.25 μl of Lipofectamine 2000 were premixed in 50 μl of OPTI-MEM (www.invitrogen.com) to generate transfection complexes that were then added to the cells. After 24 hours, the complexes were removed by replacement with fresh culture medium. The CFTR functional assay was carried out 24, 48, or 72 h after transfection. For this purpose, the cells were washed with PBS and incubated for 20 - 30 min with 60 μl PBS containing forskolin (20 μM).

After incubation, cells were transferred to a microplate reader (FluoStar Galaxy; www.bmg.labtech.com) for CFTR activity determination. The plate reader was equipped with high-quality excitation (HQ500/20X: 500 10 nm) and emission (HQ535/30M: 535 15 nm) filters for yellow fluorescent protein (www.chroma.com). Each assay consisted of a continuous 14-s fluorescence reading (5 points per second) with 2 s before and 12 s after injection of 165 μl of a modified PBS containing 137 mM Nal instead of NaCl (final NaCl concentration in the well: 100 mM). To determine iodide influx rate, the final 11 s of the data for each well were fitted with an exponential function to extrapolate initial slope.

After background subtraction, cell fluorescence recordings were normalized for the initial average value measured before addition of I-. For each well, the signal decay in the final 11 s of the data caused by YFP fluorescence quenching was fitted with an exponential function to derive the maximal slope that corresponds to initial influx of I- into the cells (Galietta et al., 2001). Maximal slopes were converted to rates of variation of intracellular I- concentration (in mM/s) using the equation:

$$d[I^-]/dt = K_I[d(F/F_0)/dt]$$

where $K_I$ is the affinity constant of YFP for I- (Galietta et al., 2001), and F/Fo is the ratio of the cell fluorescence at a given time vs. initial fluorescence.

Example 5: Animal experiments

All animal experiments were approved by the local ethics committee and carried out according to the guidelines of the German Law for the Protection of Animals (file number: 35/9185.81-2 / K/16). Cftr-/- mice (CFTRtm1Unc) were purchased from Jackson Laboratory (www.jax.org) at an age of 6- to 8-weeks and were maintained under standardized specific pathogen-free conditions on a 12 h light-dark cycle.

Food, water as well as nesting material were provided ad libitum. Prior to injections or *i.t.* spray applications mice were anesthetized intraperitoneally *(i.p.)* with a mixture of medetomidine (0.5 mg/kg), midazolam (5 mg/kg) and fentanyl (50 μg/kg). Cftr-/- mice received 40μg of hCFTR cmRNA encapsulated in chitosan-coated PLGA nanoparticles [Chitosan (83% deacetylated (Protasan UP CL 113) coated PLGA (poly-D,L-lactide-co-glycolide 75:25 (Resomer RG 752H) nanoparticles; short: NPs] by intravenous *(i.v.)* injection (n=4-3) into the tail vein and 80 μg of hCFTR cmRNA by intratracheal *(i.t.)* spraying (n=4),. Mock treated control Cftr-/- mice received 20 μg DsRed mRNA complexed to NPs (n=5) by *i.t.* delivery. For both interventions, cmRNA-NPs were administered in a total volume of 200 μl. Mice received two injections on a three day interval (day 0 and day 3). Detailed description of the i.t. procedures explained elsewhere [Mahiny et al., 2016].

After 6 days mice were sacrificed for further end point analyses. To asses immune responses to (un-)modified mRNA C57/BL6 mice (n=4 per group) were treated as described for CFTR-/- mice. As positive controls served mice that received E. coli mRNA-NPs (20μg) intravenously. C57BL/6 received one injection of 20 μg mRNA complexed to NPs. After 6 h, 24 h and 72 h mice were sacrificed and blood was collected to obtain serum.

**Example 6: Pulmonary mechanics**

Lung function for each group was evaluated using a FlexiVent equipped with FX1 module and NPFE extension and was operated by the flexiWare v7.2 software (www.scireq.com). Prior to tracheostomy, mice were anesthetized intraperitoneally as described above. After anaesthesia, a 0.5 cm incision was performed in rostral to caudal direction. A flap of skin was retracted, the connective tissue was dissected, and the trachea was exposed. The trachea was then cannulated between the second and third cartilage ring with a blunt-end stub adapter. The mouse was connected to the FlexiVent system and quasi- sinusoidally ventilated 27 with a tidal volume of 10 ml/kg. A breathing frequency of 150 breaths per min was maintained with an inspiratory to expiratory ratio of 2:3. Compliance (Cst) was calculated straight from deflating arm of the pressure volume (PV) loops and ramp style pressure-driven maneuver (PVr-P). For obtaining FEV 0.1 data a NPFE maneuver was performed which results in FV loops and FE-related parameters. The mice lung was inflated by a pressure of +30 cm $H_2O$ over 1.2 s and rapidly deflated to a negative pressure of -55 cm $H_2O$ to generate an imposed negative expiratory pressure gradient.

**Example 7: Statistics**

All analyses were performed using the Wilcoxon-Mann-Whitney test with Graphpad Prism Version 6 (www.graphpad.com). Data are represented as mean ± SD and P < 0.05 (two-sided) was considered statistically significant.

**Example 8: cmRNA-mediated expression and function of hCFTR in vitro**

To evaluate the influence of chemical nucleoside modification on hCFTR mRNA, the inventors first conducted a set of *in vitro* analyses to characterize the efficacy and functionality of hCFTR protein expression.
First, the inventors compared the expression profile of plasmid-encoded hCFTR, unmodified hCFTR mRNA carrying a reporter gene *(mKate2),* a cmRNA with N1-methylpseudo-UTP and 5-methyl-CTP as a positive control, and four nucleoside modifications with N4-acetyl-CTP of different concentrations (Fig. 1A).
The N4-acetyl-CTP cmRNAs were:

$$cmRNA^{mKate2}\frac{hCFTR}{meN1\Psi\geq1.0/m5C\geq1.0};$$

$$cmRNA^{mKate2}\frac{hCFTR}{ac4C\geq0.05};$$

$$\text{cmRNA}^{\text{mKate2}}_{\frac{hCFTR}{ac4C\geq0.25}} \text{;}$$

$$\text{cmRNA}^{\text{mKate2}}_{\frac{hCFTR}{ac4C\geq0.5}} \text{;}$$ and $\text{cmRNA}^{\text{mKate2}}$.

It turned out that N4-acetyl-CTP itself did not improve the expression as compared to a cmRNA with N1-methylpseudo-UTP and 5-methyl-CTP which was reported in the art before (Kormann 2018).

However, in a further test a cmRNA where all UTP and CTP were exchanged with N1-methylpseudo-UTP and 5-methyl-CTP cmRNA

$$\left(\text{cmRNA}^{\frac{hCFTR}{meN1\Psi\geq1.0/m5C\geq1.0}}\right) \text{ was com-}$$

pared with cmRNA where all UTP and CTP were exchanged with N1-methylpseudo-UTP and 4-acetyl-CTP

$$\left(\text{cmRNA}^{\frac{hCFTR}{meN1\Psi\geq1.0/ac4C\geq1.0}}\right).$$

The expression of

$$\text{cmRNA}^{\frac{hCFTR}{meN1\Psi\geq1.0/ac4C\geq1.0}} \text{ turned out to be}$$

3-fold higher than a control with non-modified RNA and still 2-fold higher when compared with the positive control

$$\text{cmRNA}^{\frac{hCFTR}{meN1\Psi\geq1.0/m5C\geq1.0}}.$$

Thus, it surprisingly turned out that although N4-acetyl-CTP itself did not improve the total expression, and N1-Pseudo-Uridine alone only increased the expression only to some degree. However, the expression significantly improved when N4-acetyl-CTP was combined with N1-Pseudo-Uridine (see figures 1a and 4), which resulted also in improved lung function in vivo (see figures 3 and 5).

**Example 9: YFP-assay**

To test for proper function of the cmRNA-encoded CFTR channel, the inventors utilized CFTR deficient (ΔF508 mutated) CFBE41o-cells which stably express halide-sensitive yellow fluorescent protein (YFP-H148Q/I152L). When adding iodine to the cells, the substance will be transported by existing CFTR channels into the cell. In this case the iodine is quenching the YFP, thereby decreasing its fluorescence signal. Thus, the intensity of the fluorescence signal is inversely correlated with hCFTR function. Fig. 2 shows the quenching efficacy after transfection of 250 ng/500 ng Uridine-depleted pDNA with full length hCFTR, UD-

$$\text{cmRNA}^{\frac{hCFTR}{meN1\Psi\geq1.0/ac4C\geq1.0}} \text{ and control}$$

RNA into those cells.

$$\text{UD-cmRNA}^{\frac{hCFTR}{meN1\Psi\geq1.0/ac4C\geq1.0}} \text{ shows a}$$

similar YFP signal as the Uridine-depleted pDNA with full length hCFTR.

**Sequences**

SEQ ID No.1: Coding DNA sequence ("CDS") of hCFTR:
hCFTR was PCR amplified from pcDNA3.hCFTR with primers adding *Nhe*I (Fwd: 5'-TTAGCTA-GATGCAGAGGTCGCCTC-3') and *Kpn*I (Rev: 5'-GCGGGTACCTATCTTGCATCTCTTCT -3') restriction sites to each end. The PCR product was cloned into a poly(A)-120 containing pVAX (pVAX.A120 from life technologies) by sticky-end ligation using the mentioned restriction sites.

SEQ ID No.2: mRNA sequence of hCFTR:

SEQ ID No.3: DNA sequence of hCFTR fwd primer: hCFTR fwd 5'-GAGATGCTCCTGTCTCCTGG-3'

SEQ ID No.4: DNA sequence of hCFTR rev primer: rev 5-CCTCTCCCTGCTCAGAATCT-3'

SEQ ID No.5: DNA sequence of optimal determined house-keeping gene 18S rRNA

fwd primer: 18S rRNA fwd 5'-GGGAGCCTGAGAAACGGC-3'

SEQ ID No.6: DNA sequence of optimal determined house-keeping gene 18S rRNA

rev primer: rev 5'-GACTTGCCCTCCAATGGATCC-3'

**Claims**

1. Chemically modified mRNA *(cmRNA)* with an increased stability encoding a *cystic fibrosis transmembrane conductance regulator (CFTR),* or a derivative thereof;
   wherein said chemical modification is a replacement of at least 10% of the total number of UTP and CTP nucleotides with pseudo-UTP ($\Psi U$) and acetylated CTP (acC).

2. The chemically modified mRNA of claim 1, wherein the increased stability results in an increased expression of the CFTR gene-product in a cell.

3. The chemically modified mRNA of claims 1 or 2, wherein the CFTR cmRNA is complexed with a nanoparticle, preferably a polylactic-coglycolic acid (PLGA) based nanoparticle, preferably at least partially coated with chitosan.

4. The chemically modified mRNA of any of the preceding claims configured for inhalative (p.i.) administration or an intravenous (i.v.) administration.

5. The chemically modified mRNA of any of the preceding claims, wherein said *cystic fibrosis transmembrane conductance regulator* is of human origin (hCFTR).

6. The chemically modified mRNA of claim 5, wherein at least approx. 25 %, preferably at least approx. 50%, preferably at least approx. 75%, and preferably up to including approx. 100 % of the uridine nucleotides and/or wherein at least approx. 25 %, preferably at least approx. 50%, preferably at least approx. 75%, and preferably up to approx. 100 % of the cytidine nucleotides are replaced, wherein the uridine is replaced with pseudo-UTP ($\Psi$U), preferably with N1-methylpseudo-UTP (N1$\Psi$U) and the cytidine is replaced with acetylated CTP (acC), further preferably with N4-acetyl-CTP (ac4C).

7. The chemically modified mRNA of claim 6, wherein at least approx. 50% and up to approx. 100% of UTP was replaced with N1-methylpseudo-UTP and at least approx. 25% and up to approx. 75% of CTP was replaced with N4-acetyl-CTP resulting in a chemically modified RNA abbreviated as

$$cmRNA^{hCFTR}_{N1\Psi\geq 0.5/ac4C\geq 0.5}.$$

8. The chemically modified mRNA of claims 6 or 7, wherein at least approx. 95% of UTP was replaced with N1-methylpseudo-UTP and at least approx. 50% of CTP was replaced with N4acetyl-CTP resulting in a chemically modified RNA abbreviated as

$$cmRNA^{hCFTR}_{N1\Psi\geq 0.95/ac4C\geq 0.5}.$$

9. The chemically modified mRNA of any of the preceding claims,
wherein the expression profile is increased *in vitro* by at least a factor of 2.5 as compared to the expression of an unmodified CFTR mRNA as control; and/or
wherein the expression profile is increased *in vitro* by at least a factor of 1.25 as compared to the expression of a modified CFTR cmRNA as control, with the same amount of modified UTPs and CTPs, wherein the control CFTR cmRNA comprises a methyl-CTP, such as 5-methyl-CTP.

10. The chemically modified mRNA of claim 9, wherein the expression profile is increased *in vitro* by at least a factor of 3 as compared to the expression of an unmodified CFTR mRNA as control; and/or wherein the expression is increased in vitro by at least a factor of 1.5 as compared to the expression of cmRNA with the same amount of modified UTPs and CTPs, wherein the control CFTR cmRNA comprises a methyl-CTP, such as 5-methyl-CTP ; and/or wherein the expression of CFTR

$$cmRNA^{hCFTR}_{N1\Psi 1.0/ac4C1.0}$$ is increased *in vitro* by at least a factor of 2 as compared to the expression of $$cmRNA^{hCFTR}_{N1\Psi 1.0/5mC1.0}.$$

11. The chemically modified mRNA of any of the preceding claims, wherein the CFTR cmRNA is further optimized by uridine-depletion, resulting in the increase of the expression profile in vitro by at least a factor of 4 as compared to the expression of an unmodified CFTR mRNA as control.

12. The chemically modified mRNA of any of the preceding claims, wherein 24 h after treatment of the CTFR-deficient mice with said chemically modified mRNA the Forced Expiratory Volume in 0.1 seconds (FEV0.1) in CTFR-deficient mice is at least 80% as compared with the FEV0.1 of a control group consisting of wild-type mice treated with placebo.

13. Chemically modified mRNA of any of the previous claims for use as a medicament in the treatment of a subject having or suspected of having a disease associated with the CFTR gene, preferably selected from the group consisting of: cystic fibrosis (CF), congenital absence of the vas deferens (CAVD) and/or chronic obstructive lung disease (COPD).

14. Chemically modified mRNA (cmRNA) of the claims 1 to 12 for use in the treatment of a disease associated with the CFTR gene.

15. Pharmaceutical composition comprising the chemically modified mRNA (cmRNA) according to claims 1 to 12 and further comprising a pharmaceutically acceptable excipient and/or carrier and/or diluents.

Figure 1

Figure 2

Figure 3

EP 4 085 932 A1

Figure 4

Figure 5

Europäisches Patentamt

European Patent Office

Office européen des brevets

# EUROPEAN SEARCH REPORT

Application Number

EP 21 17 1830

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/175129 A1 (ROY ATANU [US] ET AL) 22 June 2017 (2017-06-22) * para. [1535-1536],[1533],[1144]; Table 4; Tables B20, B21 * | 1-15 | INV. A61K48/00 C12N15/11 C12N15/85 |
| X | US 2014/155474 A1 (BANCEL STEPHANE [US] ET AL) 5 June 2014 (2014-06-05) * para. [1619] ,[0821-0823,1261]; Examples 71-71, 76-77, 79, 90 and 103 and tables embedded therein. * | 1-15 | |
| X | US 2017/348415 A1 (HOGE STEPHEN G [US] ET AL) 7 December 2017 (2017-12-07) * Whole doc., in partic. para. [0334 (incl. Table 8)], [0733] * | 1-15 | |
| X | SRIRAM VAIDYANATHAN ET AL: "Uridine Depletion and Chemical Modification Increase Cas9 mRNA Activity and Reduce Immunogenicity without HPLC Purification", MOLECULAR THERAPY-NUCLEIC ACIDS, vol. 12, 30 June 2018 (2018-06-30), pages 530-542, XP55531206, US ISSN: 2162-2531, DOI: 10.1016/j.omtn.2018.06.010 * abstract * | 11 | TECHNICAL FIELDS SEARCHED (IPC) A61K C12N |

The present search report has been drawn up for all claims

| Place of search | Date of completion of the search | Examiner |
|---|---|---|
| Munich | 7 January 2022 | Roscoe, Richard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................................

& : member of the same patent family, corresponding document

EPO FORM 1503 03.82 (P04C01)

Europäisches
Patentamt

European
Patent Office

Office européen
des brevets

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

**see sheet B**

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

22

Europäisches
Patentamt

European
Patent Office

Office européen
des brevets

## LACK OF UNITY OF INVENTION
## SHEET B

Application Number

**EP 21 17 1830**

The Search Division considers that the present European patent application does not comply with the requirements of unity of invention and relates to several inventions or groups of inventions, namely:

```
1. claims: 7, 8(completely); 1-6, 9-15(partially)

   Subject-matter relating to CTFR mRNA molecules which have
   been modified to increase stability by modification of at
   least 10% of the UTP nucleotides, optionally with additional
   modification of CTP nucleotides.
                          ---


2. claims: 1-6, 9-15(all partially)

   Subject-matter relating to CTFR mRNA molecules which have
   been modified to increase stability by modification of at
   least 10% of the CTP nucleotides, but no UTP nucleotides
   have been modified.
                          ---
```

## ANNEX TO THE EUROPEAN SEARCH REPORT
## ON EUROPEAN PATENT APPLICATION NO.

EP 21 17 1830

This annex lists the patent family members relating to the patent documents cited in the above-mentioned European search report.
The members are as contained in the European Patent Office EDP file on
The European Patent Office is in no way liable for these particulars which are merely given for the purpose of information.

07-01-2022

| Patent document cited in search report | Publication date | Patent family member(s) | | Publication date |
|---|---|---|---|---|
| US 2017175129 A1 | 22-06-2017 | EP | 3157572 A2 | 26-04-2017 |
| | | US | 2017175129 A1 | 22-06-2017 |
| | | WO | 2015196130 A2 | 23-12-2015 |
| US 2014155474 A1 | 05-06-2014 | AU | 2013243834 A1 | 30-10-2014 |
| | | AU | 2013243946 A1 | 30-10-2014 |
| | | AU | 2013243947 A1 | 30-10-2014 |
| | | AU | 2013243948 A1 | 30-10-2014 |
| | | AU | 2013243950 A1 | 30-10-2014 |
| | | AU | 2013243951 A1 | 30-10-2014 |
| | | AU | 2013243952 A1 | 30-10-2014 |
| | | AU | 2013243953 A1 | 30-10-2014 |
| | | AU | 2013243954 A1 | 30-10-2014 |
| | | AU | 2013243955 A1 | 30-10-2014 |
| | | AU | 2017232121 A1 | 12-10-2017 |
| | | AU | 2018200373 A1 | 22-03-2018 |
| | | AU | 2018200375 A1 | 22-03-2018 |
| | | AU | 2018200377 A1 | 22-03-2018 |
| | | AU | 2018200379 A1 | 22-03-2018 |
| | | AU | 2018200380 A1 | 22-03-2018 |
| | | AU | 2018200381 A1 | 01-02-2018 |
| | | AU | 2018247318 A1 | 08-11-2018 |
| | | AU | 2018260928 A1 | 06-12-2018 |
| | | AU | 2019202835 A1 | 16-05-2019 |
| | | AU | 2020257150 A1 | 19-11-2020 |
| | | CA | 2868393 A1 | 10-10-2013 |
| | | CA | 2868398 A1 | 10-10-2013 |
| | | CA | 2868418 A1 | 10-10-2013 |
| | | CA | 2868422 A1 | 10-10-2013 |
| | | CA | 2868429 A1 | 10-10-2013 |
| | | CA | 2868434 A1 | 10-10-2013 |
| | | CA | 2868438 A1 | 10-10-2013 |
| | | CA | 2868440 A1 | 10-10-2013 |
| | | CA | 2868996 A1 | 10-10-2013 |
| | | CA | 2869005 A1 | 10-10-2013 |
| | | CN | 104870022 A | 26-08-2015 |
| | | CN | 112390871 A | 23-02-2021 |
| | | DE | 18200782 T1 | 21-10-2021 |
| | | DE | 18203666 T1 | 07-10-2021 |
| | | EP | 2833892 A2 | 11-02-2015 |
| | | EP | 2833894 A1 | 11-02-2015 |
| | | EP | 2833920 A2 | 11-02-2015 |
| | | EP | 2833921 A2 | 11-02-2015 |
| | | EP | 2833922 A2 | 11-02-2015 |
| | | EP | 2833923 A1 | 11-02-2015 |
| | | EP | 2834259 A1 | 11-02-2015 |

EPO FORM P0459

For more details about this annex : see Official Journal of the European Patent Office, No. 12/82

page 1 of 4

## ANNEX TO THE EUROPEAN SEARCH REPORT
## ON EUROPEAN PATENT APPLICATION NO.

EP 21 17 1830

This annex lists the patent family members relating to the patent documents cited in the above-mentioned European search report.
The members are as contained in the European Patent Office EDP file on
The European Patent Office is in no way liable for these particulars which are merely given for the purpose of information.

07-01-2022

| Patent document cited in search report | Publication date | Patent family member(s) | | Publication date |
|---|---|---|---|---|
| | | EP | 2834260 A1 | 11-02-2015 |
| | | EP | 2834358 A2 | 11-02-2015 |
| | | EP | 2847329 A1 | 18-03-2015 |
| | | EP | 2849799 A2 | 25-03-2015 |
| | | EP | 3501550 A1 | 26-06-2019 |
| | | EP | 3505176 A1 | 03-07-2019 |
| | | EP | 3520820 A1 | 07-08-2019 |
| | | EP | 3520821 A1 | 07-08-2019 |
| | | HK | 1206601 A1 | 15-01-2016 |
| | | HK | 1206635 A1 | 15-01-2016 |
| | | HK | 1206636 A1 | 15-01-2016 |
| | | HK | 1206638 A1 | 15-01-2016 |
| | | HK | 1206639 A1 | 15-01-2016 |
| | | HK | 1206748 A1 | 15-01-2016 |
| | | HK | 1206779 A1 | 15-01-2016 |
| | | HK | 1206780 A1 | 12-02-2016 |
| | | HK | 1207306 A1 | 29-01-2016 |
| | | JP | 6189415 B2 | 30-08-2017 |
| | | JP | 6348482 B2 | 27-06-2018 |
| | | JP | 6424156 B2 | 14-11-2018 |
| | | JP | 6426217 B2 | 21-11-2018 |
| | | JP | 6449356 B2 | 09-01-2019 |
| | | JP | 6553104 B2 | 31-07-2019 |
| | | JP | 6946384 B2 | 06-10-2021 |
| | | JP | 6953135 B2 | 27-10-2021 |
| | | JP | 6953135 B6 | 24-11-2021 |
| | | JP | 6971953 B2 | 24-11-2021 |
| | | JP | 2015513912 A | 18-05-2015 |
| | | JP | 2015513913 A | 18-05-2015 |
| | | JP | 2015513914 A | 18-05-2015 |
| | | JP | 2015513916 A | 18-05-2015 |
| | | JP | 2015516143 A | 08-06-2015 |
| | | JP | 2015517995 A | 25-06-2015 |
| | | JP | 2015518704 A | 06-07-2015 |
| | | JP | 2015518816 A | 06-07-2015 |
| | | JP | 2015519040 A | 09-07-2015 |
| | | JP | 2015519881 A | 16-07-2015 |
| | | JP | 2017121239 A | 13-07-2017 |
| | | JP | 2017121240 A | 13-07-2017 |
| | | JP | 2017121241 A | 13-07-2017 |
| | | JP | 2017121243 A | 13-07-2017 |
| | | JP | 2017123847 A | 20-07-2017 |
| | | JP | 2017123853 A | 20-07-2017 |
| | | JP | 2017141230 A | 17-08-2017 |
| | | JP | 2017197545 A | 02-11-2017 |
| | | JP | 2018023373 A | 15-02-2018 |

EPO FORM P0459

For more details about this annex : see Official Journal of the European Patent Office, No. 12/82

**page 2 of 4**

## ANNEX TO THE EUROPEAN SEARCH REPORT
## ON EUROPEAN PATENT APPLICATION NO.

EP 21 17 1830

This annex lists the patent family members relating to the patent documents cited in the above-mentioned European search report.
The members are as contained in the European Patent Office EDP file on
The European Patent Office is in no way liable for these particulars which are merely given for the purpose of information.

07-01-2022

| Patent document cited in search report | Publication date | Patent family member(s) | | Publication date |
|---|---|---|---|---|
| | | JP | 2019033757 A | 07-03-2019 |
| | | JP | 2019054813 A | 11-04-2019 |
| | | JP | 2019107022 A | 04-07-2019 |
| | | JP | 2019216733 A | 26-12-2019 |
| | | JP | 2020072670 A | 14-05-2020 |
| | | JP | 2020158508 A | 01-10-2020 |
| | | JP | 2021045163 A | 25-03-2021 |
| | | US | 2013259923 A1 | 03-10-2013 |
| | | US | 2013259924 A1 | 03-10-2013 |
| | | US | 2014010861 A1 | 09-01-2014 |
| | | US | 2014105964 A1 | 17-04-2014 |
| | | US | 2014105965 A1 | 17-04-2014 |
| | | US | 2014105966 A1 | 17-04-2014 |
| | | US | 2014107189 A1 | 17-04-2014 |
| | | US | 2014113959 A1 | 24-04-2014 |
| | | US | 2014141067 A1 | 22-05-2014 |
| | | US | 2014148502 A1 | 29-05-2014 |
| | | US | 2014155472 A1 | 05-06-2014 |
| | | US | 2014155473 A1 | 05-06-2014 |
| | | US | 2014155474 A1 | 05-06-2014 |
| | | US | 2014155475 A1 | 05-06-2014 |
| | | US | 2014161873 A1 | 12-06-2014 |
| | | US | 2014171485 A1 | 19-06-2014 |
| | | US | 2014179771 A1 | 26-06-2014 |
| | | US | 2014186432 A1 | 03-07-2014 |
| | | US | 2014193482 A1 | 10-07-2014 |
| | | US | 2014194494 A1 | 10-07-2014 |
| | | US | 2014200262 A1 | 17-07-2014 |
| | | US | 2014200263 A1 | 17-07-2014 |
| | | US | 2014200264 A1 | 17-07-2014 |
| | | US | 2014206755 A1 | 24-07-2014 |
| | | US | 2014221465 A1 | 07-08-2014 |
| | | US | 2014249208 A1 | 04-09-2014 |
| | | US | 2014255467 A1 | 11-09-2014 |
| | | US | 2014255468 A1 | 11-09-2014 |
| | | US | 2015044277 A1 | 12-02-2015 |
| | | US | 2016075733 A1 | 17-03-2016 |
| | | US | 2017348436 A1 | 07-12-2017 |
| | | US | 2017368200 A1 | 28-12-2017 |
| | | US | 2018311381 A1 | 01-11-2018 |
| | | US | 2019240351 A1 | 08-08-2019 |
| | | US | 2019314527 A1 | 17-10-2019 |
| | | US | 2020155706 A1 | 21-05-2020 |
| | | US | 2021077634 A1 | 18-03-2021 |
| | | US | 2021299278 A1 | 30-09-2021 |
| | | WO | 2013151663 A1 | 10-10-2013 |

EPO FORM P0459

For more details about this annex : see Official Journal of the European Patent Office, No. 12/82

**page 3 of 4**

## EP 4 085 932 A1

## ANNEX TO THE EUROPEAN SEARCH REPORT
## ON EUROPEAN PATENT APPLICATION NO.

EP 21 17 1830

This annex lists the patent family members relating to the patent documents cited in the above-mentioned European search report.
The members are as contained in the European Patent Office EDP file on
The European Patent Office is in no way liable for these particulars which are merely given for the purpose of information.

07-01-2022

| Patent document cited in search report | Publication date | Patent family member(s) | | Publication date |
|---|---|---|---|---|
| | | WO | 2013151664 A1 | 10-10-2013 |
| | | WO | 2013151665 A2 | 10-10-2013 |
| | | WO | 2013151667 A1 | 10-10-2013 |
| | | WO | 2013151668 A2 | 10-10-2013 |
| | | WO | 2013151669 A1 | 10-10-2013 |
| | | WO | 2013151670 A2 | 10-10-2013 |
| | | WO | 2013151671 A1 | 10-10-2013 |
| | | WO | 2013151672 A2 | 10-10-2013 |
| | | WO | 2013151736 A2 | 10-10-2013 |
| US 2017348415 A1 | 07-12-2017 | EP | 3188749 A1 | 12-07-2017 |
| | | US | 2017348415 A1 | 07-12-2017 |
| | | WO | 2016036902 A1 | 10-03-2016 |

EPO FORM P0459

For more details about this annex : see Official Journal of the European Patent Office, No. 12/82

page 4 of 4

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015052133 A
- WO 2018202884 A1, Kormann

- WO 2011012316 A

**Non-patent literature cited in the description**

- **KORMANN et al.** Expression of therapeutic proteins after delivery of chemically modified mRNA in mice. *Letters to Nature Biotechnology,* 2011, 1-6
- **MAHINY et al.** In vivo genome editing using nuclease-encoding mRNA corrects SP-B deficiency. *Nat. Biotechnology,* 2015, vol. 33 (6), 584-586
- **DANHIER et al.** PLGA-based nanoparticles: An overview of biomedical applications. *Journal of Controlled Release,* 2012, vol. 161 (2), 505-522

- **SAMBROOK ; RUSSELL.** Molecular Cloning: A Laboratory Manual. CSH Press, Cold Spring Harbor, 2001
- Methods in Yeast Genetics, A Laboratory Course Manual. Cold Spring Harbor Laboratory Press, 1990